# EUROPEAN PATENT SPECIFICATION

(11) **EP 2 416 713 B1**
(45) Date of publication and mention of the grant of the patent: **31.05.2017**
(21) Application number: 10762401.7
(22) Date of filing: 07.04.2010
(51) Int. Cl.: A61B 17/32, A61B 17/00, A61F 9/007

(54) **DISPOSABLE HANDHELD PHACOMORCELLATION DEVICE**
TRAGBARE EINWEGVORRICHTUNG FÜR PHAKOMORCELLIERUNG
DISPOSITIF PORTATIF JETABLE DE PHACOMORCELLEMENT

(30) Priority: 07.04.2009 US 167492 P
(43) Date of publication of application: 15.02.2012
(73) Proprietor: Doheny Eye Institute, Los Angeles, CA 90033 (US)
(72) Inventor: LUE, Jaw-Chyng, Lormen, Los Angeles CA 90033 (US); MCCORMICK, Matthew, Los Angeles CA 90033 (US); KERNS, Ralph, Los Angeles CA 90033 (US)
(74) Representative: Moore, Barry
(86) International application number: PCT/US2010/030296
(87) International publication number: WO 2010/118172

(56) References cited:
- EP-A1- 0 514 057
- WO-A1-94/02075
- US-A- 4 940 468
- US-A- 5 084 052
- US-A- 5 084 052
- US-A- 5 346 497
- US-A- 5 464 389
- US-A- 5 746 713
- US-A1- 2007 191 758

## Description

### FIELD

Embodiments of the invention are generally directed to disposable bio-tissue cutter technology, and more particularly to systems and devices for facilitating the cutting and removal of bio-tissue.

### BACKROUND

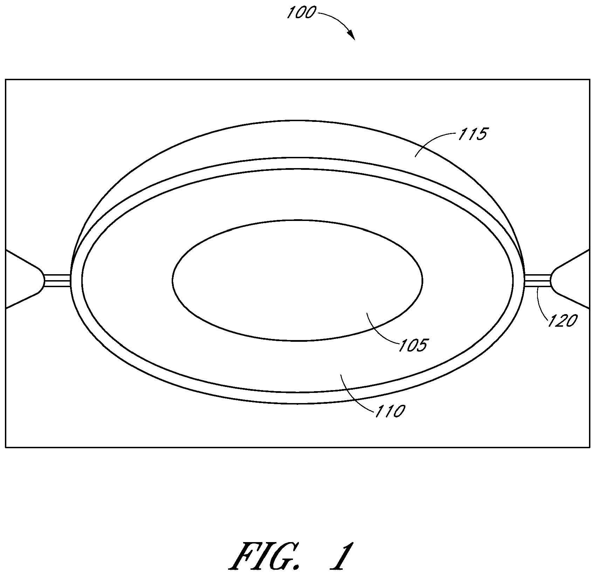

Ophthalmic surgery often involves removal of native eye tissue. One example of ophthalmic surgery that generally involves the removal of eye tissue is cataract surgery, which for most cataracts, requires removal of the native lens and replacement of the native lens with an artificial intraocular lens. Figure 1 illustrates the general anatomical structure of a native human lens 100. The lens 100 is divided into three different portions: the nucleus 105, the cortex 110, and the capsule 115. The central nucleus 105 comprises hard dense tissue and the cortex 110 comprises soft tissue. The capsule 115 is a thin transparent membrane that surrounds the cortex 110. The lens 100 is suspended behind the iris by zonula fibers 120 that connect the lens to the ciliary body.

There are a number of procedures and devices that have been developed for the removal of native lens tissue. Presently, phacoemulsification is a widely used method for removal of diseased or damaged natural lens tissue. The phacoemulsification process generally involves insertion of a probe through a small corneal incision to ultrasonically break apart and remove the native lens. EP0514057 is representative of the prior art

### SUMMARY

Various embodiments of the present invention relate to phacomorcellation devices, and systems for cutting and removing eye tissue (e.g. lens fragments) during ophthalmic surgery. The phacomorcellation devices disclosed herein are configured to prevent lens fragments from floating to a posterior region or portion of an eye. In some embodiments, the phacomorcellation device comprises a stationary outer tubular cutting member having a proximal end and a distal end, the proximal end coupled to a housing, the distal end having a first opening, the first opening having a first cutting edge and a first point formed by two intersecting arcs, the first point positioned on a first side of the first opening. The phacomorcellation device can further comprise a motor positioned within the housing and selectively controllable by one or more user control inputs coupled to the housing. The phacomorcellation device can also comprise an inner cutting member having a proximal end and a distal end, the inner cutting member positioned within the stationary outer tubular cutting member, the motor coupled to the proximal end to rotate the inner cutting member relative to the stationary outer tubular cutting member, the inner cutting member having a second opening that is substantially symmetrical to the first opening, the second opening having a second cutting edge and a second point formed by two intersecting arcs, wherein the first opening is substantially closed when the inner cutting member is rotated into a closed position.

In various embodiments the inner cutting member comprises a helical bit having an outer diameter that is less than an inner diameter of the outer tubular cutting member. In various embodiments, the outer diameter at the distal end of the helical bit and the inner diameter of the outer tubular cutting member have a ratio of less than 0.8, and the outer diameter of an intermediate portion of the helical bit proximal to the distal end and the inner diameter of the outer tubular cutting member have a ratio of greater than 0.8. In some embodiments, the outer diameter at the distal end of the helical bit and the inner diameter of the outer tubular cutting member have a ratio of less than 0.7, and the outer diameter of an intermediate portion of the helical bit proximal to the distal end and the inner diameter of the outer tubular cutting member have a ratio of greater than 0.7. In some embodiments, the outer diameter at the distal end of the helical bit and the inner diameter of the outer tubular cutting member have a ratio of less than 0.5, and the outer diameter of an intermediate portion of the helical bit and the inner diameter of the outer tubular cutting member have a ratio of greater than 0.5.

In various embodiments, the phacomorcellation device further comprises an aspiration chamber within the housing and an aspiration line coupled to the aspiration chamber configured to remove lens fragments from the surgical site through the outer tubular cutting member and the inner cutting member. In various embodiments, the phacomorcellation device comprises an outer sleeve configured to surround a portion of the outer tubular cutting member, the outer sleeve having a distal opening for the first opening. In various embodiments, the outer sleeve comprises silicone. The outer sleeve can further comprise one or more openings, ports, or apertures configured to deliver irrigation to the surgical site.

In various embodiments, the inner cutting member of the phacomorcellation device is configured to oscillate longitudinally while rotating within the outer tubular cutting member. In some embodiments, the outer tubular cutting member comprises a distal tip surface and a side wall surface, wherein the first opening is formed partially within the distal tip surface and the side wall surface. The distal tip surface can comprise a planar surface that extends substantially perpendicular to a longitudinal axis of the phacomorcellation device. In various embodiments, the first point formed by the two intersecting arcs of the first opening is coplanar with the planar distal tip surface.

A method of using a phacomorcellation device to prevent lens fragments from floating to a posterior portion of an eye during surgery comprises accessing a surgical site with a stationary outer tubular cutting member; receiving tissue within an opening positioned at a distal end of the outer tubular cutting member, the opening having a first cutting edge and a first point formed by two intersecting arcs, the first point positioned on a first side of the first opening; engaging the tissue with the first point to prevent the tissue from floating away from the phacomorcellation device; and rotating the inner cutting member positioned within the outer tubular cutting member to sever the tissue, the inner cutting member having a second cutting edge, and a second point formed by two intersecting arcs, the second opening is substantially symmetrical to the first opening, the second point positioned on an opposite side relative to the first opening, wherein the first opening is substantially closed when the inner cutting member is rotated into a closed position.

A method can further comprise oscillating longitudinally the inner cutting member to further sever the tissue. A method comprises irrigating the surgical site with irrigation fluid and aspirating the surgical site to remove tissue and fluid from the surgical site.

According to various embodiments, a phacomorcellation device configured to prevent lens fragments from floating to a posterior portion of an eye comprises a stationary outer tubular cutting member having a proximal end and a distal end, the proximal end coupled to a housing, the distal end having a first port, the first port having a first cutting edge and a second cutting edge and a first pointed end member formed by the intersection of the first and second cutting edges, the first point positioned on a first side of the first port. The phacomorcellation device can further comprise a motor positioned within the housing and selectively controllable by one or more user control inputs coupled to the housing. The phacomorcellation device can also comprise an inner cutting member having a proximal end and a distal end, the inner cutting member positioned within the stationary outer tubular cutting member, the motor coupled to the proximal end to rotate the inner cutting member relative to the stationary outer tubular cutting member, the inner cutting member having a second port that is substantially symmetrical to the first port, the second port having a third cutting edge and a fourth cutting edge and a second pointed end member formed by the intersection of the third and fourth cutting edges. In various embodiments, the first port can be substantially closed when the inner cutting member is rotated into a closed position.

In various embodiments, a phacomorcellation surgical instrument configured to prevent lens fragments from floating to a posterior portion of an eye comprises a first stationary elongate tubular member having a distal end and a proximal end, the proximal end coupled to a housing, the distal end having a first opening, the first opening having a first cutting edge and a first point formed by two intersecting arcs, the first point positioned on a first side of the first opening. The phacomorcellation surgical instrument can also comprise a motor positioned within the housing and controllable by user inputs coupled to the housing. In various embodiments, the phacomorcellation surgical instrument further comprises a second elongate member having a proximal end and a distal end, the second elongate member positioned within the first stationary elongate tubular member and the first opening, the motor coupled to the proximal end to rotate the second elongate member relative to the stationary elongate tubular member, the distal end having a second opening that is substantially symmetrical to the first opening, the second opening having a second cutting edge and a second point formed by two intersecting arcs, the second point positioned on an opposite side relative to the first opening, wherein the first opening is substantially closed when the second elongate member is rotated into a closed position.

According to various embodiments, a phacomorcellation device configured to remove lens fragments from a surgical site of an eye comprises a stationary outer tubular cutting member having a proximal end and a distal end, the proximal end coupled to a housing, the distal end having a distal cutting port defined within an end surface of the distal end, the distal cutting port having a first cutting edge and a radial side cutting port defined within a wall surface at the distal end, the radial side cutting port having a second cutting edge. The phacomorcellation device can also comprise a motor positioned within the housing and selectively controllable by one or more user control inputs coupled to the housing and an inner cutting member having a proximal end and a distal end, the inner cutting member positioned within the stationary outer tubular cutting member, the motor coupled to the proximal end to rotate the inner cutting member relative to the stationary outer tubular cutting member, the inner cutting member having a third cutting edge. In various embodiments, the third cutting edge of the inner cutting member is cooperable with the second cutting edge of the stationary outer tubular cutting member to form a bird beak cutting structure configured to grasp and cut lens fragments of the eye, and an opening defined between the third cutting edge of the inner cutting member and the second cutting edge of the outer cutting member is substantially closed during rotation of the inner cutting member.

A method of using a phacomorcellation device to prevent lens fragments from floating to a posterior portion of an eye during ophthalmic surgery comprises accessing a surgical site with a cutting tip of a phacomorcellation device, the cutting tip comprising a stationary outer tubular cutting member and an inner cutting member positioned concentrically within the outer tubular cutting member and configured to rotate within the stationary outer tubular cutting member; receiving lens tissue within a cutting port positioned at a distal end of the outer tubular cutting member, the distal cutting port having a first arcuate cutting edge on a distal end surface of the distal end and a second arcuate cutting edge on a side wall surface of the distal end and a first point member formed by the intersection of the first and second arcuate cutting edges; engaging the lens tissue with the first point member to prevent the tissue from floating away from the phacomorcellation device; and rotating the inner cutting member positioned within the outer tubular cutting member to sever the tissue, the inner cutting member having a third arcuate cutting edge. In various embodiments, the second arcuate cutting edge of the outer tubular cutting member and the third arcuate cutting edge form a bird beak cutting structure configured to grasp and cut the lens tissue, and an opening defined between the second arcuate cutting edge and the third arcuate cutting edge is substantially closed during rotation of the inner cutting member with respect to the tubular outer cutting member.

In various embodiments, a phacomorcellation device comprises a cylindrical outer tubular cutter that has a beak with two sharp curved edges near its distal end, with one curved edge on the cylindrical wall surface and the other curved edge on the distal end surface. The cylindrical outer tubular cutter can be connected to a housing at its proximal end such that it remains stationary. The phacomorcellation device also comprises an inner cutter positioned concentrically within the outer tubular cutter that has a beak with two sharp curved edges near its distal end and an optional helical blade, with one curved edge on the cylindrical wall surface and the other curved edge on the distal end surface. The helical blade can be continuous or non-continuous from the beak structure end all the way to an aspiration. The inner cutter can rotate within the outer tubular cutter.

For purposes of this summary, certain aspects, advantages, and novel features of the invention are described herein. It is to be understood that not necessarily all such aspects, advantages, and features may be employed and/or achieved in accordance with any particular embodiment of the invention. Thus, for example, those skilled in the art will recognize that the invention may be embodied or carried out in a manner that achieves one advantage or group of advantages as taught herein without necessarily achieving other advantages as may be taught or suggested herein.The application is defined by the claims.

### BRIEF DESCRIPTION OF THE DRAWINGS

The foregoing and other features, aspects and advantages of the present invention are described in detail below with reference to the drawings of various embodiments, which are intended to illustrate and not to limit the invention. The drawings comprise the following figures in which:
Figure 1 illustrates the general anatomical structure of a native human lens.
Figure 2 is a cross-sectional view of an embodiment of a handheld morcellation device.
Figures 3A-3D illustrate a distal cutting end of an embodiment of a handheld morcellation device.
Figures 3E-3J illustrate the cutting operation of an embodiment of a handheld morcellation device as an inner cutting member is rotated within an outer cutting member.
Figures 4A and 4B illustrate cutting profiles of the inner cutting member and the outer cutting member of an embodiment of a symmetrical-type cutting tip.
Figures 5A and 5B illustrate perspective views of a symmetrical-type cutting tip of an embodiment of a handheld morcellation device.
Figures 6A and 6B illustrate cutting profiles of the inner cutting member and the outer cutting member of an embodiment of an asymmetrical-type cutting tip.
Figure 7 illustrates the general anatomy of the eye and an example surgical entry location for insertion of a cutting tip of a handheld morcellation device within the eye.

### DETAILED DESCRIPTION OF THE PREFERRED EMBODIMENT

Embodiments of the invention will now be described with reference to the accompanying figures, wherein like numerals refer to like elements throughout. The terminology used in the description presented herein is not intended to be interpreted in any limited or restrictive manner, simply because it is being utilized in conjunction with a detailed description of certain specific embodiments of the invention. Furthermore, embodiments of the invention may comprise several novel features, no single one of which is solely responsible for its desirable attributes or which is essential to practicing the inventions herein described.

The embodiments herein illustrate handheld morcellation devices that are portable, disposable, robust, low-power, cost effective, and can morcellate and/or remove bio-tissue from a patient. The term "morcellation" means the breaking up of bio-tissue into smaller pieces. The term "phacomorcellation" can be used to describe the breaking up of lens tissue into smaller pieces for removal (for example, during cataract surgery), as the prefix "phaco-" means lens. Embodiments of the phacomorcellation devices described herein can advantageously be configured to prevent lens fragments from being projected toward a posterior portion of the eye, thereby preventing potential damage to the retina and other posterior eye structures.

Although embodiments of the invention will generally be described in conjunction with cataract surgery, it is the Applicants' intention that embodiments of the invention could be modified for use in other fields or applications as well, such as removing cartilage, muscle, ligament, tendon, or bone tissue during orthopedic surgery. In certain embodiments, the morcellation devices described herein can be used to remove lenses or lens fragments that have dropped into the vitreous or posterior regions of the eye.

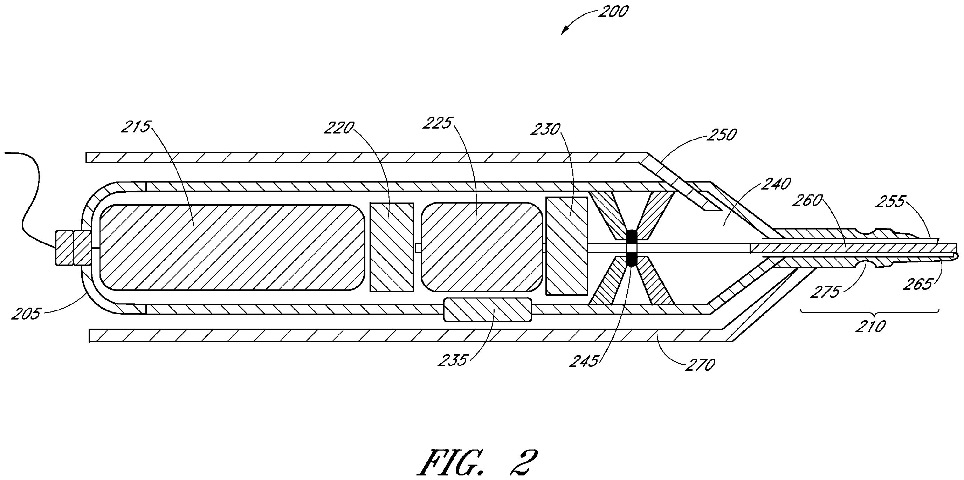

Figure 2 illustrates a cross-sectional view of an embodiment of a disposable handheld morcellation device 200. It should be appreciated that Figure 2 is a schematic drawing and the individual components are not necessarily to scale. The morcellation device 200 comprises a housing 205 and a cutting tip 210.

The housing 205 encapsulates the internal components of the morcellation device 200 and allows the surgeon to grasp and manipulate the morcellation device during surgery. In some embodiments, the morcellation device 200 is configured for single-handed operation. The length of the housing 205 can be less than about 130 mm and the outer diameter of the housing 205 can be less than about 17 mm. In one embodiment, the length of the housing 205 is about 70 mm and the outer diameter of the housing 205 is about 15 mm.

The cutting tip 210 can be configured to cut, emulsify, and/or remove bio-tissue. The length of the cutting tip 210 can range from about 5 mm to about 150 mm, as desired and/or required for various types of surgical procedures. In one embodiment, the length of the cutting tip 210 is about 20 mm.

The internal components of the housing 205 can comprise a power supply 215, a control/drive circuit 220, a motor 225, a gear set box 230, one or more control inputs 235, and an aspiration chamber 240. In one embodiment, the housing optionally comprises one or more structural elements (e.g., an O-ring) that form a vacuum seal 245 above the aspiration chamber 240 within the handheld device 200. The aspiration chamber 240 can function as a reservoir of waste fluid for potential reflux purposes. An aspiration line 250 can be coupled to the aspiration chamber 240 to remove the broken-up pieces of bio-tissue during operation of the morcellation device 200. In some embodiments, the inner diameter of the aspiration chamber 240 can be less than about 20 mm and the length of the aspiration chamber can be less than about 15 mm; however, these dimensions can be varied as desired and/or required.

In some embodiments, the vacuum aspiration function provided by the aspiration chamber 240 facilitates the grasping and holding of the tissue fragments to improve the cutting and removal of the bio-tissue. In other embodiments, the housing 205 does not comprise a vacuum sealed aspiration chamber or aspiration line.

In some embodiments, the power supply 215 comprises a battery or other internal power supply (e.g., a rechargeable or disposable battery, one or more capacitors, one or more ultracapacitors, and/or the like). In various embodiments, the internal power supply comprises a battery ranging from 1-15 Volts (e.g., 6V, 9V, 12V, 15V). The handheld device can additionally or alternatively be electrically coupled to an external power source via a power connector disposed at a proximal end (opposite the distal cutting end) of the housing.

The control/drive circuit 220 can be powered by the power supply 215 and can be configured to control the operation of the motor 225, which in turn drives gears in the gear box 230 to effect rotation of an actuator. In some embodiments, the control/drive circuit can comprise a circuit board and one or more electronic devices coupled to the circuit board. The circuit board can be contained within a housing unit. The circuit board and/or housing unit can be sized and shaped to fit within the internal diameter of the housing 205. In some embodiments, the dimensions of the control/drive circuit board and/or housing unit can be less than about 20 mm (height) by less than about 16 mm (length) by less than about 16 mm (width); however, other dimensions can be used as desired and/or required.

The transmission gear box 230 can be used to transition a relatively higher speed and lower torque rotary motion to a lower speed yet higher torque motion (thus stronger force) for cutting hard tissues (for example, nucleus, cataract, cartilage). The transmission gear box 230 can advantageously increase the torque output of the motor 225 without requiring a high speed motor. In certain embodiments, the dimensions of the transmission gear box can be less than about 15 mm (height) by less than about 16 mm (length) by less than about 16 mm (width); however, other dimensions can be used as desired and/or required.

In one embodiment, the motor comprises a rotary motor; however, other types of motors are contemplated. For example, the motor 225 can be used to effect rotary motion, circular or linear oscillation, and/or vibration, of the cutting tip 210. In various embodiments, the motor can cause the cutting tip 210 to oscillate longitudinally while the cutting tip 210 is rotating. In some embodiments, the dimensions of the motor can be less than about 30 mm (height) by less than about 16 mm (length) by less than about 16 mm (width); however, other dimensions can be used as desired and/or required.

In embodiments wherein rotary motion is employed, the handheld morcellation device 200 can be advantageously designed to rotate using high torque at low speeds. Typical rotary cutters run at high speeds (for example, > 1000 RPM), which can easily cause fluid swirling in the subtle anterior chamber during surgery. Swirling fragments of broken-up bio-tissue (for example, lens fragments) can impact subtle eye regions and cause unwanted damage. By adopting a much lower speed (for example, < about 200 RPM), the fragmentation procedure becomes gentler and the swirling effect is greatly diminished, thereby reducing the possibility of unwanted damage. In some embodiments, the cutting speed of the morcellation device can range from about 10 RPM to about 1000 RPM; however, other cutting speeds can be used as desired and/or required for various procedures and/or types of bio-tissue. The lower speeds can advantageously reduce or prevent the occurrence of fragments being projected toward the posterior eye region at high speeds during removal of eye tissue by the morcellation device 200, thereby preventing damage to the posterior eye structures.

Although the handheld morcellation device 200 is designed to run at a low rotation speed, increased torque can be generated by the transmission gear set box 230. For example, the transmission gear set box 230 can provide a 10:1 transition in certain embodiments. In various embodiments, the transmission gear set box 230 can provide a range of transitions from 1:1 to 100:1 as desired and/or required. As a consequence of the increased torque provided by the gear set box 230, more force can be applied to the bio-tissue (e.g., cataract and/or nucleus) using low power. Accordingly, the handheld morcellation device 200 can advantageously reduce the need for high-powered devices. In some embodiments, the handheld morcellation device 200 operates using a standard 9V power supply instead of a high-power 40V power supply.

In some embodiments, the torque range of the cutting tip 210 after gear transmission can range from about 50 mN-m to about 10,000 mN-m, depending on the type of surgery being performed and the type of tissue being cut. For example, when used for cataract surgery, the torque range can span from about 50 mN-m to about 1000 mN-m. The particular torque used can be configured based on the radius of the inner cutting member to result in a force of up to about 20 N for cutting the cataract. For example, a 100 mN-m torque can be used for an inner cutting member having a 0.5 mm radius. When used for orthopedic surgery, the torque range can span from about 500 mN-m to about 10,000 mN-m. The particular torque used can be configured based on the radius of the inner cutting member to result in a force of up to about 100 N for cutting cartilage tissue. For example, a 6000 mN-m torque can be used for an inner cutting member having a 0.6 cm radius.

The control inputs 235 can, for example, toggle the power on and off, vary the cutting speed or rotation rate of the cutting tip 210, and/or toggle aspiration on and off or adjust aspiration levels. The control inputs 235 can comprise switches, buttons, touch-sensitive elements, and/or other input devices. In some embodiments, the control inputs 235 are configured for single-hand operation using one or more fingers on the hand that is holding the morcellation device 200. The control inputs 235 can be positioned at any position on the exterior of the housing 205 or accessible from the exterior of the housing 205. In some embodiments, a first control input is used to power on and off the morcellation device 200 and a second control input is used to activate the cutting tip (e.g., rotation, oscillation, and/or vibration).

The illustrated cutting tip 210 comprises a tubular outer cutting member 255 and an inner cutting member 260 positioned concentrically within the tubular outer cutting member 255. In some embodiments, the inner cutting member 260 is not attached to the tubular outer cutting member 255. The tubular outer cutting member 255 can be coupled to a distal end of the housing 205. In one embodiment, the tubular outer cutting member 255 remains stationary as the inner cutting member 260 rotates within the tubular outer cutting member 255. In other embodiments, the outer cutting member 255 moves with respect to the inner cutting member 260. In still other embodiments, both the outer cutting member 255 and the inner cutting member 260 rotate in opposite directions.

The outer diameter of the outer cutting member 255 can range from about 0.5 mm to about 15 mm. For example, the outer diameter of the outer cutting member 255 in morcellation devices used for cataract surgery can range from about 0.5 mm to about 2.5 mm and the outer diameter of the outer cutting member 255 in morcellation devices used for orthopedic surgery can range from about 1 mm to about 20 mm. The outer diameter of the inner cutting member 260 can be any dimension that is at least about 0.1 mm smaller than the dimension of the outer diameter of the outer cutting member 255. In certain embodiments, the outer diameter of the inner cutting member 260 ranges from about 10% to about 100% (e.g., 10%, 20%, 30%, 40%, 50%, 60%, 70%, 80%, 90%) of the inner diameter of the outer cutting member 255. The outer diameter or maximum cross-sectional dimension of the inner cutting member 260 can vary along its length. For example, the maximum cross-sectional dimension at the distal tip of the inner cutting member 260 can be smaller than the maximum cross-sectional dimension of a region proximal to the distal tip of the inner cutting member 260, thereby increasing the ability of the morcellation device 200 to grasp and hold relatively large lens fragments within the cutting tip 210 for further breakdown.

Optionally, as shown in Figure 2, a soft outer sleeve 265 can be placed over at least a portion of the cutting tip 210. In some embodiments, the outer sleeve 265 comprises an asymmetric sleeve structure that partially surrounds the outer cutting member 255, wherein the sleeve structure comprises one or more cutout portions that leave open the major cutting region of the cutting tip 210. In one embodiment, an irrigation line 270 can be coupled to the cutting tip 210 through the outer sleeve in order to allow for irrigation during use. The outer sleeve 265 can include one or more apertures, openings, or ports 275 through which irrigation fluid can be delivered to the surgical site. For example, the irrigation line 270 can be employed during cataract surgery. In other embodiments, irrigation is not required and/or desired.

The outer sleeve 265 can comprise a soft protective tip disposed around the cutting tip 210 to prevent rupture of the lens capsule during cataract surgery. The soft outer sleeve 265 can function as an isolation wall between the delicate regions (for example, the posterior lens capsule) and the cutting tip of the cutting tip 210. In certain embodiments, the soft outer sleeve 265 can have a thickness between about 0.1 mm and about 1 mm. The soft outer sleeve 265 can be configured to cover any portion of the length of the cutting tip 210 or the entire length of the cutting tip 210 (while leaving the one or more cutting surfaces exposed). The outer sleeve 265 can comprise one or more polymeric materials, such as silicone.

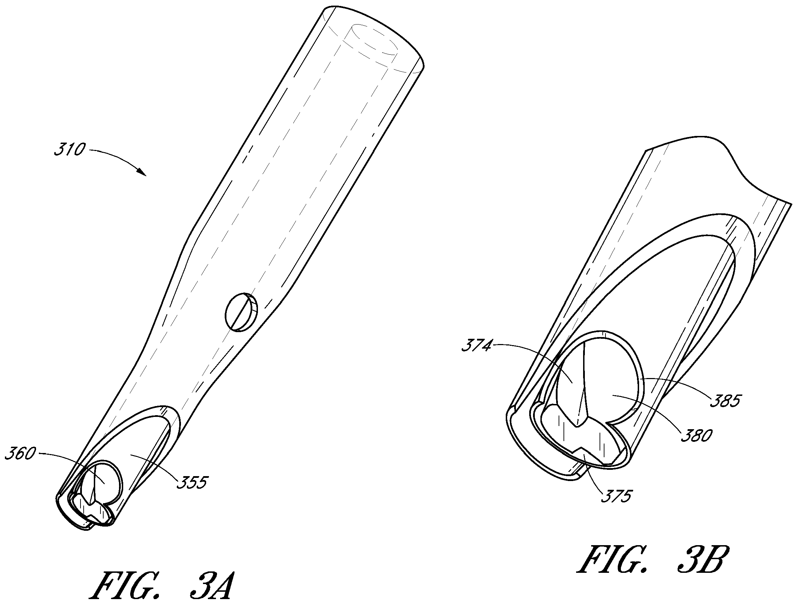

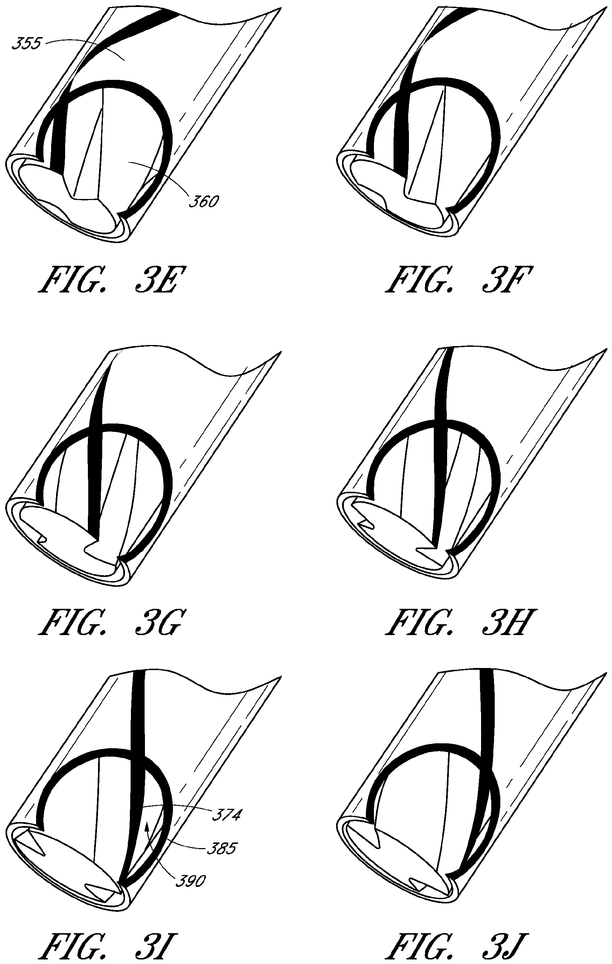

Figure 3A illustrates a perspective view of an embodiment of a cutting tip 310 of the handheld morcellation device 200. Figure 3B illustrates an enlarged perspective view of the distal end of the cutting tip 310. As shown, the cutting tip 310 comprises a helical inner cutting member 360, a cylindrical tubular outer cutting member 355, and an outer sleeve 365. In some embodiments, the tubular outer cutting member 355 remains stationary while the helical inner cutting member 360 rotates. The rotation of only one of the cutting members can advantageously reduce or prevent the occurrence of lens fragments being projected from the distal end of the cutting tip 210 toward the posterior region of the eye and/or reduce the speed at which the lens fragments are projected toward the posterior region of the eye.

The helical inner cutting member 360 comprises a rotary helical bit with two end-milled helical blades 374. The tubular outer cutting member 355 comprises a distal cutting port 375 at the distal end surface and a radial side cutting port 380 formed within a cylindrical wall surface at the distal end of the tubular outer cutting member 355. The distal cutting port 375 and the radial side cutting port 380 can be formed in the shape of truncated circles, the truncated portions of which intersect each other perpendicularly to form two pointed cusps at the intersections of the truncated circles. The cusps can be formed by the intersection of two arcs (for example, two generally arcuate cutting edges or bird-beak blades). In various embodiments, the cutting ports 375, 380 can comprise one or more openings, cutouts, apertures, or recesses of any shape. For example, the cutting ports 375, 380 form a single, integral opening. The radial side cutting port 380 comprises a sharp curved cutting edge that defines a second bird beak blade 385. In some embodiments, the distal edge of the distal cutting port 375 is sharp (e.g., beveled). In some embodiments, the distal tips of the distal ends of the inner cutting member 360 and the tubular outer cutting member 355 are blunt (for example, so as not to rupture the lens capsule during cataract surgery).

The distal cutting port 375 can make it such that not only protruded tissues but also flat tissues can be clutched, enclosed and thus cut by the morcellation device 200. The side cutting port 380 can allow for morcellation with enhanced visualization by the operator. In some embodiments, the morcellation device 200 comprises either a distal cutting port 375 or a side cutting port 380 and not both. In other embodiments, the distal cutting port 375 and the side cutting port 380 are not integrally connected as illustrated by the figures herein, but comprise separate stand-alone ports or openings.

In general, the term "bird beak" as used herein shall be given its ordinary meaning and shall refer, without limitation, to a sharp beak-like (tooth-like) structure that, in cooperation with one or more other beak-like structures approaching bio-tissue from a substantially opposite direction, forms a "beak-like," or wedge-shaped, cutting structure capable of exerting sufficient force on focused areas of unwanted bio-tissue in order to morcellate the bio-tissue.

In one embodiment, the inner cutting member 360 can be coupled to an actuator, which effects rotary movement of the inner cutting member 360. One of ordinary skill in the art will appreciate that other bits, such as guillotine-type bits may be used as the inner cutting member 360 with the embodiments without deviating from the scope of the disclosure.

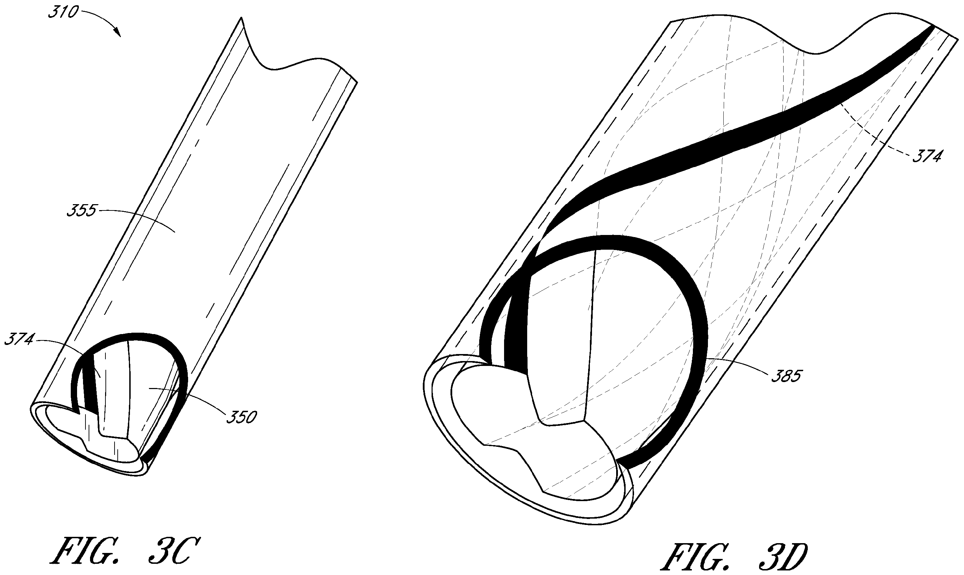

Figure 3C illustrates a close-up perspective view of the distal end of the cutting tip 310 (outer sleeve 365 not shown). Figure 3D illustrates an outline view of the helical inner cutting member 360 inside the tubular outer cutting member 355. As discussed above, the helical inner cutting member 360 comprises two end-milled helical blades 374. The helical inner cutting member 360 can comprise one or more bird beak blades on its distal end surface. The sharp curved edge (e.g., bird beak blade) 385 of the radial side cutting port 380, or opening, of the tubular outer cutting member 355 and the end-milled helical blades 374 of the helical inner cutting member 360 cooperate with each other to comprise the major cutting portions of the cutting tip 310. The helical blades 374 of the helical inner cutting member 360 can be continuous or non-continuous from the beak structure at the distal end of the helical inner cutting member 360 up to the aspiration chamber 240.

Figures 3E-3J illustrate six continuous "snapshots" of enlarged views of the cutting tip 310 during rotation of the helical inner cutting member 360 within the stationary tubular outer cutting member 355. The major cutting blades are highlighted by bolded lines. As shown in Figures 3E-3J, as the helical inner cutting member 360 rotates within the stationary tubular outer cutting member 355, the cutting blades of the helical inner cutting member 360 and the stationary tubular outer cutting member 355 cooperate to form a "bird-beak," or wedge-shaped, cutting structure 390. In some embodiments, a bird-beak cutting structure is formed at the distal cutting port 375 and/or the side cutting port 380. Because of the design of the curved slope of the bird beak edges, bio-tissue can be pulled into the beak structure's mouth once acquired. The aspiration line can aid in pulling the bio-tissue into the cutting tip and holding the bio-tissue until it is cut. The bio-tissue can be broken up bit by bit, thereby preventing lens fragments from floating or otherwise being expelled from the cutting ports 375, 380.

During cutting of the hard nucleus of the lens, the bird beak cutting structure can be extremely efficient because the forces the beak blades exert on the tissue are focused. In addition, the bird-beak cutting structure formed by the combination of the blades or cutting edges of the tubular outer cutting member 355 and the helical inner cutting member 360 enable the cutting tip 310 to clutch, pinch, and cut flat tissues, as well as protruded tissues, with or without use of a vacuum.

The tubular outer cutting member 355 remains stationary, as shown in Figures 3E-3J, during rotation of the helical inner cutting member 360. When the two sharp "beaks" of the bird-beak cutting structure grab and then anchor the bio-tissue, the pressure at the contact points can build up quickly as the rotary motion continues. The sharp edges on the cylindrical wall surfaces of the tubular outer cutting member 355 and the helical inner cutting member 360 function as a pair of small "scissors" responsible for the circumferential cut. The sharp edges on the distal end surfaces function as another pair of "scissors" responsible for the cut along the distal end plane. The wedge-shaped structure formed by the combination of the edges causes the bio-tissue to be pulled up into the morcellation device 200 to be further broken down before it enters the aspiration chamber 240.

The bird beak cutting structures can advantageously prevent lens fragments from floating or being projected toward the posterior region of the eye. In the initial "biting" phases of morcellation, the local pressures at the contact points between the beaks and the bio-tissue are relatively high (pressure = force/area) in order to effect the pinching, shearing, cutting, or biting action. If the bio-tissue is brittle, this initial action can crack the bio-tissue. If the bio-tissue is relatively soft, the beak structure's sharp blade edges can pull and shear cut the bio-tissue after the initial pinching action.

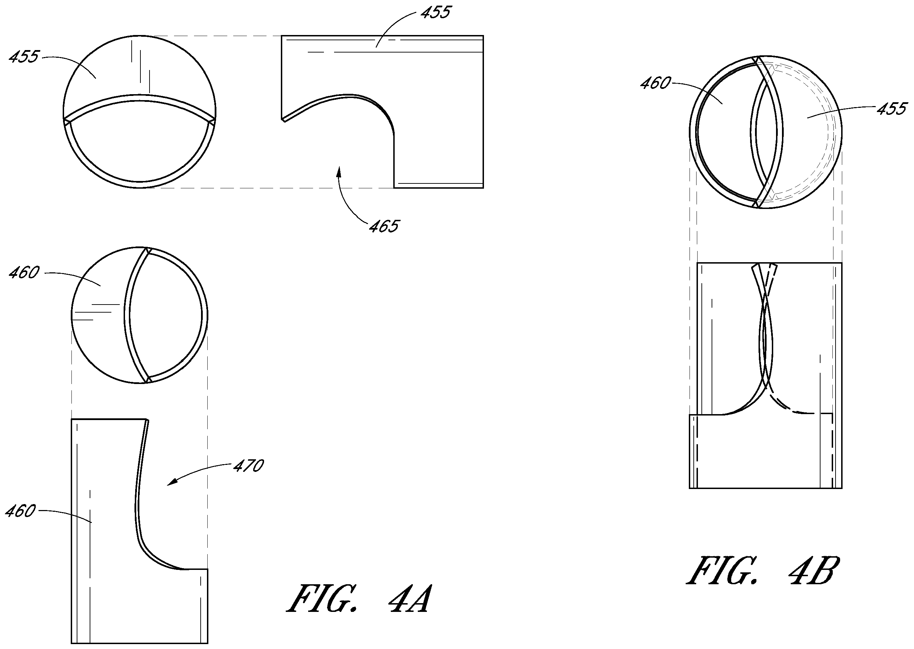

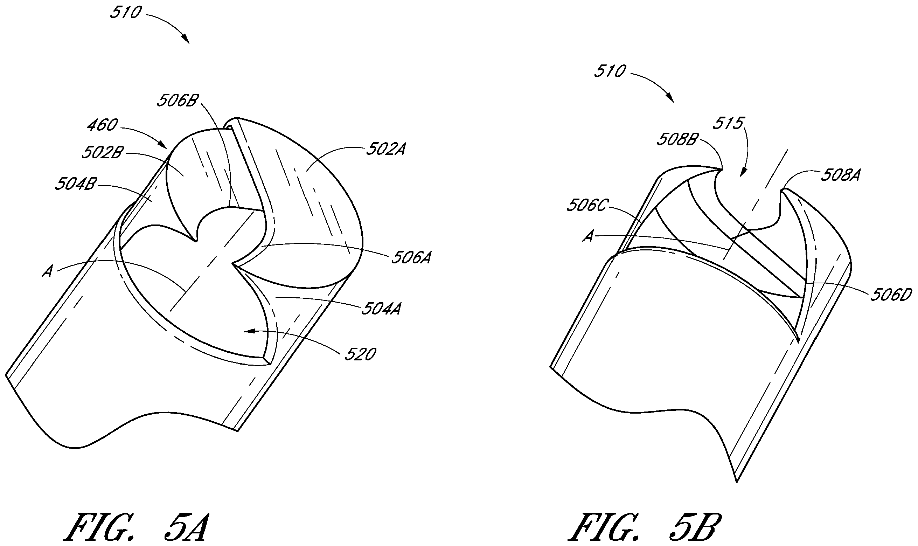

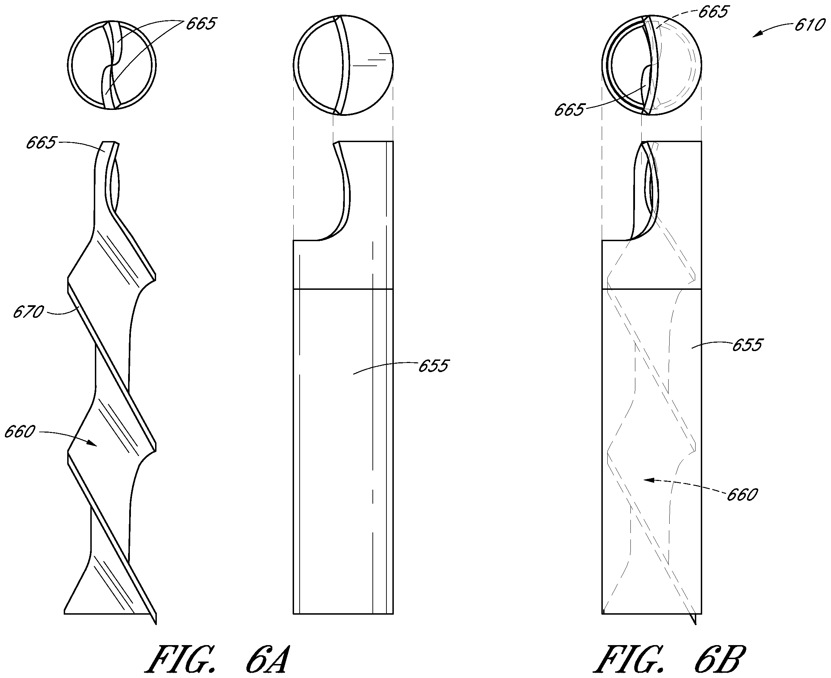

It should be appreciated that various embodiments of the inner cutting member 260 and the tubular outer cutting member 255 can be designed to create various embodiments of combination bird-beak cutting tips. Various alternative embodiments are illustrated in Figures 4A, 4B, 5A, 5B, 6A and 6B. Figures 4A, 4B, 5A and 5B illustrate symmetrical-type bird-beak cutting tips suitable for rotary and/or oscillatory motion cutting. Figures 6A and 6B illustrate an asymmetrical-type bird-beak cutting tip suitable for rotary motion cutting.

Figure 4A illustrates side and end views of the distal ends of a tubular outer cutting member 455 and an inner cutting member 460. The tubular outer cutting member 455 and the inner cutting member 460 together comprise a symmetrical-type cutting tip of a morcellation device. Figure 4B illustrates side and end views of the symmetrical-type cutting tip formed by the combination of the tubular outer cutting member 455 and the inner cutting member 460. The distal end surfaces and radial side wall surfaces of the outer cutting member 455 and the inner cutting member comprise generally concave beak blades formed by scooped-out recesses, or openings, in the distal end surface and the side wall of the outer cutting member 455 and the inner cutting member 460. For example, the outer cutting member 455 can comprise a first opening 465 at its distal end and the inner cutting member 460 can comprise a second opening 470 at its distal end. The generally concave beak blades terminate in points, or pointed end members, at the intersection of the beak blades on the distal end and the side wall of the outer cutting member 455 and the inner cutting member 460. The shapes of the beak blades and the scooped-out recesses, or openings, can vary as desired and/or required. The beak blades can be beveled or otherwise sharpened to provide cutting edges. The curved beak blades of the outer cutting member 455 and the inner cutting member 460 cooperate to form bird beak cutting structures as the beak blades overlap during rotation of the inner cutting member 460 with respect to the outer cutting member 455, as shown in Figure 4B.

Figures 5A and 5B illustrate perspective views of a symmetrical-type cutting tip 510. The bird beak cutting structures formed by the combination of a stationary outer elongate tubular member (e.g., the tubular outer cutting member 455) and a rotatable inner elongate member (e.g., the inner cutting member 460) are symmetrical about a central longitudinal axis A of the phacomorcellation device. As shown in Figures 5A and 5B, first and second bird-beak cutting structures are formed on the distal end surfaces 502A, 502B and on the radial side wall surfaces 504A, 504B, respectively, of the cutting tip 510. The distal end surfaces 502 comprise flat, planar, or substantially planar surfaces that are perpendicular or substantially perpendicular to the longitudinal axis A of the phacomorcellation device. In some embodiments, the flat, planar, or substantially planar distal end surfaces advantageously prevent rupture of a capsule during cataract surgery or damage to surrounding eye tissue that is not intended to be removed. The distal end surfaces 502 and radial side wall surfaces 504 of the outer cutting member 455 and the inner cutting member 460 comprise generally concave beak blades 506A-506D formed by scooped-out portions, or openings, in the distal end surfaces 502 and/or the side wall surfaces 504 of the outer cutting member 455 and the inner cutting member 460. The scooped-out portion of the outer cutting member 455 forms a first opening, or port. The scooped-out portion of the inner cutting member 460 forms a second opening, or port. The second opening of the inner cutting member 460 can be substantially symmetrical to the first opening of the outer cutting member 455.

The generally concave beak blades 506 terminate in points, or pointed end members, 508A, 508B at the intersection of the beak blades 506. In some embodiments, the points 508 are formed by two intersecting arcs (e.g., two of the concave beak blades 506). In some embodiments, one or both of the points 508A, 508B are coplanar with the distal end surfaces 502 of the outer cutting member 455 and the inner cutting member 460. In other embodiments, one or more of the points 508A, 508B are offset from the plane of the distal end surfaces 502. The shapes of the beak blades 506 and the scooped-out portions can vary as desired and/or required. The beak blades 506 can be beveled or otherwise sharpened to provide cutting edges. In some embodiments, at least a portion of the beak blades 506 are curved or arcuate.

The first opening, or port, at the distal end of the tubular outer cutting member 455 can be substantially closed when the inner cutting member 460 is rotated into a closed position. More particularly, as the inner cutting member 460 rotates within the outer tubular cutting member 455, openings formed between the arcuate bird-beak blades 506B, 506C of the inner cutting member 460 and the cooperating arcuate bird-beak blades 506A, 506D of the tubular outer cutting member 455 are substantially closed. As the respective cooperating bird-beak blades 506 approach the substantially closed position during rotation of the inner cutting member 460, the openings formed between the respective cooperating bird beak blades 506 transition from a semicircular shape to an almond shape. For example, a distal opening 515 formed between the arcuate cutting edges of the beak blades 506A, 506B on the distal end surfaces 502 of the tubular outer cutting member 455 and the inner cutting member 460 is substantially closed in at least one rotation position of the inner cutting member 460. As the distal opening 515 is closed, the beak blades 506A, 506B can cut the tissue. A radial side opening 520 formed between the arcuate cutting edges of the beak blades 506C, 506D of the radial side wall surfaces 504 of the tubular outer cutting member 455 and the inner cutting member 460 can be substantially closed in at least one rotation position of the inner cutting member 460.

The substantial closure of the distal and radial side openings 515, 520 can advantageously aid in reducing or preventing the drifting, rejection, or expulsion of lens or other tissue fragments from the morcellation device towards the posterior region of the eye, thereby reducing the likelihood of damage to the eye. As shown in Figures 5A and 5B, the distal and radial side openings 515, 520 can be integrally connected to form a single overall opening in at least some of the rotation positions of the inner cutting member 460.

Figures 6A and 6B illustrate an asymmetrical-type bird-beak cutting tip 610 comprising a helical inner cutting member 660 and a tubular outer cutting member 655. Figure 6A illustrates side and end views of the helical inner cutting member 660 and the tubular outer cutting member 655 and Figure 6B illustrates a side and end view of the asymmetrical-type cutting tip formed by the combination of the tubular outer cutting member 655 and the helical inner cutting member 660. The helical inner cutting member 660 comprises one or more bird beak blades 665 at its distal tip.

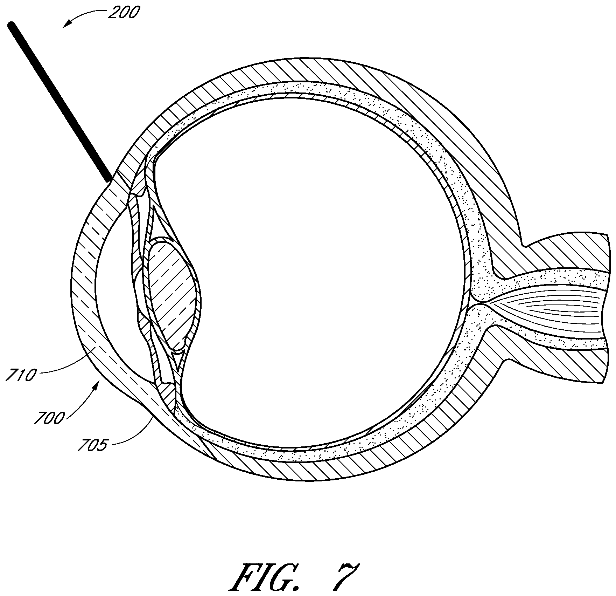

Figure 7 is a cross-sectional view of the overall anatomy of an eye 700 and illustrates an example insertion site of the handheld morcellation device 200 within the eye. In one embodiment, the cutting tip 210 of the handheld morcellation device 200 can be inserted through a small incision located at the corneo-scleral junction 705 and then though a circular hole in the lens capsule. It should be appreciated by one of ordinary skill in the art that insertion into the cornea 710 can occur anywhere near the corneo-scleral junction 705 of the eye, through the sclera alone, through the cornea alone, or at other locations of the eye. After insertion of the handheld morcellation device, the morcellation device is powered on and used to morcellate the inner nucleus through rotary and/or oscillatory motion of the inner cutting member within the outer cutting member. The nucleus is comprised of hard material that must be broken up into pieces in order to be removed without necessitating a large incision that would greatly prolong recovery time and increase risk of infection. The aspiration line 250 can be used to remove the broken-up pieces of the nucleus from the eye through. The aspiration line 250 can advantageously facilitate the grabbing and holding of the eye tissue to be cut and removed by the morcellation device 200.

Once the hard nucleus is removed, the remaining soft cortex material is removed through the aspiration line 250. In one embodiment, the cutting tip 210 can still aspirate without activating rotation of the inner cutting member 260. Therefore, surgeons have the freedom to use the pumping action that can be provided by the inner cutting member 260 or not during aspiration of soft tissues. For example, when a helical bit is used for the inner cutting member 260, the helical blade structure of the helical bit can help push and aspirate the fragments of the tissues into the handheld device 200. By increasing the rotation speed, the helical bit can provide even more effective aspiration.

The use of a helical bit for the inner cutting member 260 can advantageously aid in the prevention of fluid surge and/or occlusion of the aspiration line 250. The helical blades of the helical bit can help fragment the bio-tissues that could potentially become clogged within the critical aspiration channel, which is the channel between the helical bit and the inner wall of the tubular outer cutting member 255. The helical bit structure helps push fragments and wastes into the tubular outer cutting member 255 while rotating at a relatively high speed, and prevents unwanted damages on tissues such as posterior capsule due to clogs and consequential vacuum surges in the tubular cutter tip during surgery.

As illustrated in Figures 6A and 6B, for example, the helical inner cutting member 660 can be designed with a relatively narrow beak-like blade 665 close to its distal tip and a drill-bit like structure 670 extending away from the distal tip of the beak-like blade. For example, the outer diameter at the distal tip of the helical inner cutting member 660 can range from about 10% to about 50% of the inner diameter of the outer cutting member 655 and the outer diameter of the drill bit portion can range from about 50% to about 100% of the inner diameter of the outer cutting member 655. The drill-bit-like structure can be used to help transport and fragment the already-aspirated fragments of bio-tissue. All the fragments that enter the handheld morcellation device must be smaller than the space between the inner wall of the outer cutting member 655 and the outer surface of the helical bit. Therefore, the opportunity of clogging the wider aspiration line 250 extending out from the aspiration chamber 240 is greatly reduced.

It should be appreciated that, in contrast to ultrasonic phacoemulsification devices, embodiments of the handheld morcellation device 200 described herein are constructed of low-cost materials such that the handheld morcellation device can be disposed of after a single surgery, thus eliminating contamination and infection risks due to repetitive use of the device without proper sterilization. In addition, embodiments of the handheld morcellation device operate at low power, which reduces the risk of overheating or burning the cornea during surgery.

The morcellation devices described herein can advantageously be used in conjunction with the surgical racks, trays, and/or centers described in the following patent applications: U.S. Patent Application Serial No. 12/107,038, filed April 21, 2008, now published as U.S. Publication No. 2008/0281254; U.S. Patent Application Serial No. 12/256,420, filed October 22, 2008, now published as U.S. Publication No. 2009/0143734; U.S. Patent Application Serial No. 12/684,850, filed January 8, 2010; U.S. Patent Application Serial No. 12/107,052, filed April 21, 2008, now published as U.S. Publication No. 2008/0281301; and U.S. Patent Application Serial No. 12/106,962, filed April 21, 2008, now published as U.S. Publication No. 2008/0272023.

Furthermore, to those skilled in the various arts, the invention itself herein will suggest solutions to other tasks and adaptations for other applications, such as orthopedic applications. It is the Applicants' intention to cover all such uses of the invention and those changes and modifications which could be made to the embodiments of the invention described herein without departing from the scope of the invention. Thus, the present embodiments of the invention should be considered in all respects as illustrative and not restrictive.

Conditional language, for example, among others, "can," "could," "might," or "may," unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without user input or prompting, whether these features, elements and/or steps are included or are to be performed in any particular embodiment.

While the invention has been discussed in terms of certain embodiments, it should be appreciated that the invention is not so limited. The embodiments are explained herein by way of example, and there are numerous modifications, variations and other embodiments that may be employed that would still be within the scope of the present invention. Some embodiments have been described in connection with the accompanying drawings. However, it should be understood that the figures are not necessarily drawn to scale. Distances, angles, etc. are merely illustrative and do not necessarily bear an exact relationship to actual dimensions and layout of the devices illustrated. Components can be added, removed, and/or rearranged. Additionally, the skilled artisan will recognize that any of the above-described methods can be carried out using any appropriate apparatus. Further, the disclosure herein of any particular feature, aspect, method, property, characteristic, quality, attribute, element, or the like in connection with various embodiments can be used in all other embodiments set forth herein. Additionally, processing steps may be added, removed, or reordered. A wide variety of designs and approaches are possible.

For purposes of this disclosure, certain aspects, advantages, and novel features of the invention are described herein. It is to be understood that not necessarily all such advantages may be achieved in accordance with any particular embodiment of the invention. Thus, for example, those skilled in the art will recognize that the invention may be embodied or carried out in a manner that achieves one advantage or group of advantages as taught herein without necessarily achieving other advantages as may be taught or suggested herein.

## Claims

1. A phacomorcellation device (200) configured to prevent lens fragments from floating to a posterior portion of an eye, the phacomorcellation device comprising:
a stationary outer tubular cutting member (255, 355, 455) having a proximal end and a distal end, the proximal end coupled to a housing (205), the distal end having a first opening;
a motor (225) positioned within the housing (205) and selectively controllable by one or more user control inputs coupled to the housing; and
an inner cutting member (260, 360, 460) having a proximal end and a distal end, the inner cutting member positioned within the stationary outer tubular cutting member (455), the motor (255) coupled to the proximal end to rotate the inner cutting member relative to the stationary outer tubular cutting member, the inner cutting member having a second opening that is substantially symmetrical to the first opening, the second opening having a second cutting edge (506B);
wherein the first opening is substantially closed when the inner cutting member (460) is rotated into a closed position, **characterised in that** the first opening further having a first cutting edge (506A) and a first point (508A) formed by two intersecting arcs (506A, 506D), the first point positioned on a first side of the first opening and the second opening further having a second cutting edge (506B) and a second point (508B) formed by two intersecting arcs (506B, 506C).

2. The phacomorcellation device of Claim 1, wherein the inner cutting member (360) comprises a helical bit having an outer diameter that is less than an inner diameter of the outer tubular cutting member (355).

3. The phacomorcellation device of Claim 2, wherein the outer diameter at a distal end of the helical bit and the inner diameter of the outer tubular cutting member (355) have a ratio of less than 0.8, and the outer diameter of an intermediate portion of the helical bit proximal to the distal end and the inner diameter of the outer tubular cutting member (355) have a ratio of greater than 0.8.

4. The phacomorcellation device of Claim 2, wherein the outer diameter at a distal end of the helical bit and the inner diameter of the outer tubular cutting member (355) have a ratio of less than 0.7, and the outer diameter of an intermediate portion of the helical bit proximal to the distal end and the inner diameter of the outer tubular cutting member (355) have a ratio of greater than 0.7.

5. The phacomorcellation device of Claim 2, wherein the outer diameter at a distal end of the helical bit and the inner diameter of the outer tubular cutting member (355) have a ratio of less than 0.5, and the outer diameter of an intermediate portion of the helical bit and the inner diameter of the outer tubular cutting member (355) have a ratio of greater than 0.5.

6. The phacomorcellation device of Claim 1, further comprising an aspiration chamber (240) within the housing (205) and an aspiration line (250) coupled to the aspiration chamber configured to remove lens fragments from the surgical site through the outer tubular cutting member (255) and the inner cutting member (260).

7. The phacomorcellation device of Claim 1, further comprising an outer sleeve (265) configured to surround a portion of the outer tubular cutting member (255), the outer sleeve having a distal opening for the first opening.

8. The phacomorcellation device of Claim 7, wherein the outer sleeve (265) comprises silicone.

9. The phacomorcellation device of Claim 8, wherein the outer sleeve (265) comprises one or more openings (275) configured to deliver irrigation to the surgical site.

10. The phacomorcellation device of Claim 1, wherein the inner cutting member (260) is configured to oscillate longitudinally while rotating within the outer tubular cutting member (255).

11. The phacomorcellation device of Claim 1, wherein the motor (225) is powered by a battery.

12. The phacomorcellation device of Claim 1, the outer tubular cutting member (455) comprises a distal tip surface (502A) and a side wall surface (504A), wherein the first opening is formed partially within the distal tip surface and the side wall surface.

13. The phacomorcellation device of Claim 12, wherein the distal tip surface (502A) comprises a planar surface that extends substantially perpendicular to a longitudinal axis (A) of the phacomorcellation device.

14. The phacomorcellation device of Claim 13, wherein the first point is coplanar with the planar distal tip surface (502A).

15. The phacomorcellation device of Claim 1, wherein the second opening comprises a third cutting edge and wherein the second point is formed by an intersection of the second and third cutting edges.

16. The phacomorcellation device of Claim 1, wherein the inner cutting member (455) further comprises a third cutting edge, the third cutting edge of the inner cutting member (455) is cooperable with the first cutting edge of the stationary outer tubular cutting member (460) to form a bird beak cutting structure configured to grasp and cut lens fragments of the eye, and
wherein an opening defined between the third cutting edge of the inner cutting member and the first cutting edge of the outer cutting member is substantially closed during rotation of the inner cutting member (460).

## Patentansprüche

1. Phacomorcellierungsvorrichtung (200), die ausgelegt ist zum Verhindern, dass Linsenfragmente zu einem posterioren Teil eines Auges treiben können, wobei die Phacomorcellierungsvorrichtung Folgendes umfasst:
ein stationäres äußeres tubusförmiges Schneidelement (255, 355, 455), das ein proximales Ende und ein distales Ende aufweist, wobei das proximale Ende mit einem Gehäuse (205) gekoppelt ist und das distale Ende eine erste Öffnung aufweist;
einen Motor (225), der innerhalb des Gehäuses (205) positioniert und selektiv von einem oder mehreren Benutzersteuerungseingängen, die mit dem Gehäuse gekoppelt sind, steuerbar ist; und
ein inneres Schneidelement (260, 360, 460), das ein proximales Ende und ein distales Ende aufweist, wobei das innere Schneidelement innerhalb des stationären äußeren tubusförmigen Schneidelements (455) positioniert ist, wobei der Motor (255) mit dem proximalen Ende gekoppelt ist, um das innere Schneidelement relativ zum stationären äußeren tubusförmigen Schneidelement zu drehen, wobei das innere Schneidelement eine zweite Öffnung aufweist, die im Wesentlichen symmetrisch zur ersten Öffnung ist, wobei die zweite Öffnung eine zweite Schneide (506B) aufweist;
wobei die erste Öffnung im Wesentlichen geschlossen ist, wenn das innere Schneidelement (460) in eine geschlossene Position gedreht ist, **dadurch gekennzeichnet, dass** die erste Öffnung ferner eine erste Schneide (506A) und einen ersten Punkt (508A) aufweist, der von zwei sich schneidenden Bögen (506A, 506D) gebildet wird, wobei der erste Punkt an einer ersten Seite der ersten Öffnung positioniert ist und die zweite Öffnung ferner eine zweite Schneide (506B) und einen zweiten Punkt (508B) aufweist, der von zwei sich schneidenden Bögen (506B, 506C) gebildet wird.

2. Phacomorcellierungsvorrichtung nach Anspruch 1, wobei das innere Schneidelement (360) einen helixförmigen Einsatz umfasst, der einen Außendurchmesser aufweist, der kleiner als ein Innendurchmesser des äußeren tubusförmigen Schneidelements (355) ist.

3. Phacomorcellierungsvorrichtung nach Anspruch 2, wobei der Außendurchmesser an einem distalen Ende des helixförmigen Einsatzes und der Innendurchmesser des äußeren tubusförmigen Schneidelements (355) ein Verhältnis von weniger als 0,8 aufweisen und der Außendurchmesser eines Zwischenteils des helixförmigen Einsatzes proximal zum distalen Ende und der Innendurchmesser des äußeren tubusförmigen Schneidelements (355) ein Verhältnis von mehr als 0,8 aufweisen.

4. Phacomorcellierungsvorrichtung nach Anspruch 2, wobei der Außendurchmesser an einem distalen Ende des helixförmigen Einsatzes und der Innendurchmesser des äußeren tubusförmigen Schneidelements (355) ein Verhältnis von weniger als 0,7 aufweisen und der Außendurchmesser eines Zwischenteils des helixförmigen Einsatzes proximal zum distalen Ende und der Innendurchmesser des äußeren tubusförmigen Schneidelements (355) ein Verhältnis von mehr als 0,7 aufweisen.

5. Phacomorcellierungsvorrichtung nach Anspruch 2, wobei der Außendurchmesser an einem distalen Ende des helixförmigen Einsatzes und der Innendurchmesser des äußeren tubusförmigen Schneidelements (355) ein Verhältnis von weniger als 0,5 aufweisen und der Außendurchmesser eines Zwischenteils des helixförmigen Einsatzes und der Innendurchmesser des äußeren tubusförmigen Schneidelements (355) ein Verhältnis von mehr als 0,5 aufweisen.

6. Phacomorcellierungsvorrichtung nach Anspruch 1, die ferner eine Absaugkammer (240) innerhalb des Gehäuses (205) und eine Absaugleitung (250), die mit der Absaugkammer gekoppelt ist, umfasst, die ausgelegt sind zum Entfernen von Linsenfragmenten von der Eingriffsstelle durch das äußere tubusförmige Schneidelement (255) und das innere Schneidelement (260).

7. Phacomorcellierungsvorrichtung nach Anspruch 1, die ferner eine äußere Hülse (265) umfasst, die ausgelegt ist zum Umgeben eines Teils des äußeren tubusförmigen Schneidelements (255), wobei die äußere Hülse eine distale Öffnung für die erste Öffnung aufweist.

8. Phacomorcellierungsvorrichtung nach Anspruch 7, wobei die äußere Hülse (265) Silikon umfasst.

9. Phacomorcellierungsvorrichtung nach Anspruch 8, wobei die äußere Hülse (265) eine oder mehrere Öffnungen (275) umfasst, die ausgelegt sind zum Liefern von Spülung an die Eingriffsstelle.

10. Phacomorcellierungsvorrichtung nach Anspruch 1, wobei das innere Schneidelement (260) ausgelegt ist zum longitudinalen Oszillieren während es innerhalb des äußeren tubusförmigen Schneidelements (255) rotiert.

11. Phacomorcellierungsvorrichtung nach Anspruch 1, wobei der Motor (225) von einer Batterie mit Strom versorgt wird.

12. Phacomorcellierungsvorrichtung nach Anspruch 1, wobei das äußere tubusförmige Schneidelement (455) eine distale Spitzenoberfläche (502A) und eine Seitenwandoberfläche (504A) aufweist, wobei die erste Öffnung teilweise innerhalb der distalen Spitzenoberfläche und der Seitenwandoberfläche gebildet ist.

13. Phacomorcellierungsvorrichtung nach Anspruch 12, wobei die distale Spitzenoberfläche (502A) eine ebene Oberfläche umfasst, die sich im Wesentlichen senkrecht zu einer Längsachse (A) der Phacomorcellierungsvorrichtung erstreckt.

14. Phacomorcellierungsvorrichtung nach Anspruch 13, wobei der erste Punkt komplanar mit der ebenen distalen Spitzenoberfläche (502A) ist.

15. Phacomorcellierungsvorrichtung nach Anspruch 1, wobei die zweite Öffnung eine dritte Schneide umfasst und wobei der zweite Punkt durch eine Überschneidung der zweiten und der dritten Schneide gebildet wird.

16. Phacomorcellierungsvorrichtung nach Anspruch 1, wobei das innere Schneidelement (455) ferner eine dritte Schneide umfasst, wobei die dritte Schneide des inneren Schneidelements (455) mit der ersten Schneide des stationären äußeren tubusförmigen Schneidelements (460) zusammenwirkt, um eine Vogelschnabelschneidstruktur zu bilden, die ausgelegt ist zum Greifen und Schneiden von Linsenfragmenten des Auges, und
wobei eine zwischen der dritten Schneide des inneren Schneidelements und der ersten Schneide des äußeren Schneidelements definierte Öffnung im Wesentlichen während des Drehens des inneren Schneidelements (460) geschlossen ist.

## Revendications

1. Dispositif de phacomorcellation (200) configuré pour empêcher des fragments de lentille de flotter vers une portion postérieure d'un oeil, le dispositif de phacomorcellation comprenant :
un organe de coupe tubulaire externe immobile (255, 355, 455) ayant une extrémité proximale et une extrémité distale, l'extrémité proximale étant couplée à un logement (205), l'extrémité distale ayant une première ouverture ;
un moteur (225) positionné au sein du logement (205) et pouvant être sélectivement commandé par une ou plusieurs entrées de commande d'utilisateur couplées au logement ; et
un organe de coupe interne (260, 360, 460) ayant une extrémité proximale et une extrémité distale, l'organe de coupe interne étant positionné au sein de l'organe de coupe tubulaire externe immobile (455), le moteur (255) étant couplé à l'extrémité proximale pour faire tourner l'organe de coupe interne par rapport à l'organe de coupe tubulaire externe immobile, l'organe de coupe interne ayant une seconde ouverture qui est sensiblement symétrique à la première ouverture, la seconde ouverture ayant une deuxième arête tranchante (506B) ;
dans lequel la première ouverture est sensiblement fermée lorsque l'organe de coupe interne (460) est tourné dans une position fermée, **caractérisé en ce que** la première ouverture comporte en outre une première arête tranchante (506A) et un premier point (508A) formé par deux arcs sécants (506A, 506D), le premier point étant positionné sur un premier côté de la première ouverture et la seconde ouverture ayant en outre une deuxième arête tranchante (506B) et un second point (508B) formé par deux arcs sécants (506B, 506C).

2. Dispositif de phacomorcellation selon la revendication 1, dans lequel l'organe de coupe interne (360) comprend une mèche hélicoïdale ayant un diamètre extérieur qui est inférieur au diamètre intérieur de l'organe de coupe tubulaire externe (355).

3. Dispositif de phacomorcellation selon la revendication 2, dans lequel le diamètre extérieur à une extrémité distale de la mèche hélicoïdale et le diamètre intérieur de l'organe de coupe tubulaire externe (355) ont un rapport de moins de 0,8, et le diamètre extérieur d'une portion intermédiaire de la mèche hélicoïdale à proximité de l'extrémité distale et le diamètre intérieur de l'organe de coupe tubulaire externe (355) ont un rapport de plus de 0,8.

4. Dispositif de phacomorcellation selon la revendication 2, dans lequel le diamètre extérieur à une extrémité distale de la mèche hélicoïdale et le diamètre intérieur de l'organe de coupe tubulaire externe (355) ont un rapport de moins de 0,7, et le diamètre extérieur d'une portion intermédiaire de la mèche hélicoïdale à proximité de l'extrémité distale et le diamètre intérieur de l'organe de coupe tubulaire externe (355) ont un rapport de plus de 0,7.

5. Dispositif de phacomorcellation selon la revendication 2, dans lequel le diamètre extérieur à une extrémité distale de la mèche hélicoïdale et le diamètre intérieur de l'organe de coupe tubulaire externe (355) ont un rapport de moins de 0,5, et le diamètre extérieur d'une portion intermédiaire de la mèche hélicoïdale et le diamètre intérieur de l'organe de coupe tubulaire externe (355) ont un rapport de plus de 0,5.

6. Dispositif de phacomorcellation selon la revendication 1, comprenant en outre une chambre d'aspiration (240) au sein du logement (205) et une ligne d'aspiration (250) couplée à la chambre d'aspiration configurée pour enlever des fragments de lentille du site chirurgical par l'intermédiaire de l'organe de coupe tubulaire externe (255) et de l'organe de coupe interne (260).

7. Dispositif de phacomorcellation selon la revendication 1, comprenant en outre un manchon externe (265) configuré pour cerner une portion de l'organe de coupe tubulaire externe (255), le manchon externe ayant une ouverture distale pour la première ouverture.

8. Dispositif de phacomorcellation selon la revendication 7, dans lequel le manchon externe (265) comprend du silicone.

9. Dispositif de phacomorcellation selon la revendication 8, dans lequel le manchon externe (265) comprend une ou plusieurs ouvertures (275) configurées pour délivrer une irrigation au site chirurgical.

10. Dispositif de phacomorcellation selon la revendication 1, dans lequel l'organe de coupe interne (260) est configuré pour osciller longitudinalement tout en tournant au sein de l'organe de coupe tubulaire externe (255).

11. Dispositif de phacomorcellation selon la revendication 1, dans lequel le moteur (225) est alimenté par une batterie.

12. Dispositif de phacomorcellation selon la revendication 1, dans lequel l'organe de coupe tubulaire externe (455) comprend une surface d'embout distal (502A) et une surface de paroi latérale (504A), dans lequel la première ouverture est formée partiellement au sein de la surface d'embout distal et de la surface de paroi latérale.

13. Dispositif de phacomorcellation selon la revendication 12, dans lequel la surface d'embout distal (502A) comprend une surface plane qui s'étend sensiblement perpendiculaire à un axe longitudinal (A) du dispositif de phacomorcellation.

14. Dispositif de phacomorcellation selon la revendication 13, dans lequel le premier point est coplanaire avec la surface d'embout distal plane (502A).

15. Dispositif de phacomorcellation selon la revendication 1, dans lequel la seconde ouverture comprend une troisième arête tranchante et dans lequel le second point est formé par une intersection des deuxième et troisième arêtes tranchantes.

16. Dispositif de phacomorcellation selon la revendication 1, dans lequel l'organe de coupe interne (455) comprend en outre une troisième arête tranchante, la troisième arête tranchante de l'organe de coupe interne (455) peut coopérer avec la première arête tranchante de l'organe de coupe tubulaire externe immobile (460) pour former une structure de coupe en bec d'oiseau configurée pour saisir et couper des fragments de lentille de l'oeil, et
dans lequel une ouverture définie entre la troisième arête tranchante de l'organe de coupe interne et la première arête tranchante de l'organe de coupe externe est sensiblement fermée pendant une rotation de l'organe de coupe interne (460).
